# EUROPEAN PATENT APPLICATION

(11) **EP 4 555 983 A1**
(43) Date of publication of application: **21.05.2025**
(21) Application number: 23839234.4
(22) Date of filing: 14.03.2023
(51) Int. Cl.: A61F 11/00

(54) **VESTIBULAR FUNCTION IMPROVEMENT DEVICE, DRUNKENNESS STIMULATION APPLICATION SYSTEM, AND PROGRAM**

(30) Priority: 13.07.2022 JP 2022112199
(71) Applicant: NATIONAL UNIVERSITY CORPORATION TOKAI NATIONAL HIGHER EDUCATION AND RESEARCH SYSTEM, Nagoya-shi, Aichi 464-8601 (JP)
(72) Inventor: KATO, Masashi, Nagoya-shi, Aichi 464-8601 (JP); OHGAMI, Nobutaka, Nagoya-shi, Aichi 464-8601 (JP)
(74) Representative: Studio Torta S.p.A.
(86) International application number: PCT/JP2023/009900
(87) International publication number: WO 2024/014055

(57) **Abstract**

A vestibular function improvement device 1 for generating a sound stimulus to improve the vestibular function of the inner ear, wherein the frequency of the sound stimulus changes over time.

## Description

### FIELD

This disclosure relates to a technology for improving vestibular function of the inner ear using sound stimulus, and in particular, to a technology for alleviating motion sickness using sound stimulus.

### BACKGROUND

The prevalence of motion sickness is very high, affecting 60% of adults, and its incidence aboard ships reaches as high as 100%. Recently, the development of autonomous driving technology for vehicles has been progressing rapidly. It has been reported that passengers are at a higher risk of motion sickness compared to drivers, especially when engaging in non-driving activities such as reading during travel, which significantly increases the risk of motion sickness (e.g., Non-Patent Document 1). Furthermore, motion sickness can also be induced by a visual stimulus such as that experienced in VR or XR environments. Against this backdrop, the development of technologies to mitigate motion sickness is highly desired.

In this regard, the present inventors have discovered that a low-frequency sound stimulus can influence the vestibular function of the inner ear and have developed a vestibular stimulation device to activate vestibular function using a sound stimulus (Patent Document 1). This device generates a sound stimulus with a constant frequency between 20 and 140 Hz (pure tone) to activate vestibular function and is expected to provide preventive and therapeutic effects for motion sickness, including car sickness, VR sickness, and related conditions.

### Related Art Documents, Patent Documents

### [Patent Document]

[Patent Document 1] International Publication No. WO 2019/230941

### [Non-Patent Document]

[Non-Patent Document 1] DiZio et al.,"An Active Suspension System for Mitigating Motion Sickness and Enabling Reading in a Car",Aerospace Medicine and Human Performance,Volume 89,Number 9,September 2018,pp. 822-829(8)
[Non-Patent Document 2] Nalivaiko et al.,"Motion sickness, nausea and thermoregulation: The "toxic" hypothesis", Temperature, 2014 Oct-Dec; 1(3): 164-171.

### SUMMARY OF THE INVENTION

### Technical Problem

However, the conventional technology described in Patent Document 1 did not clarify at which timing-before, during, or after the motion sickness-inducing stimulus-sound stimulus should be applied to achieve an improvement effect. Therefore, the present inventors conducted experiments on mice to verify the effectiveness of pure tone stimulus, as described in the conventional technology, in alleviating motion sickness.

First, the evaluation of motion sickness in mice can be assessed by measuring the tail temperature after the motion stimulus, as a decrease in tail temperature has been reported to indicate the worsening of motion sickness (Non-Patent Literature 2). To verify this, we observed the fluctuations in the tail temperature of the mice following the motion stimulus. Specifically, six mice were used: three mice (rotation stimulus group) were subjected to a 10-minute rotational motion stimulus, and three mice (linear stimulus group) were subjected to a 10-minute linear motion stimulus. Tail temperatures were measured before, during, and after the stimulus.

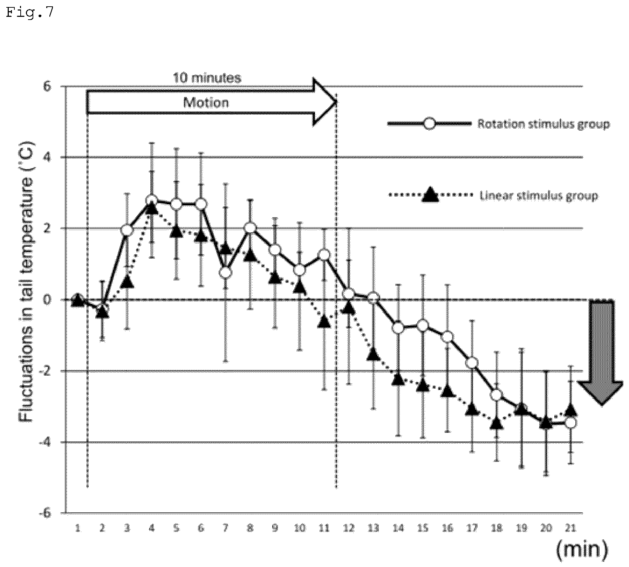

Figure 7 is a graph showing the fluctuations in the tail temperature of mice, with the temperature before the stimulus set as the baseline (zero point). In all mice, the tail temperature increased during the motion stimulus and decreased after the stimulus. This confirms that, in mice, a decrease in tail temperature after the motion stimulus indicates the onset of motion sickness.

To evaluate the relationship between the vestibular function in the inner ear and motion sickness, a motion stimulus test was conducted using VL mice (Vestibular Lesion mice), which have impaired inner ear function. Specifically, six mice were prepared, and three of them were subjected to inner ear dysfunction through the intratympanic administration of ototoxic drugs. Then, all six mice were given a 10-minute linear motion stimulus, and their tail temperatures were measured before, during, and after the stimulus.

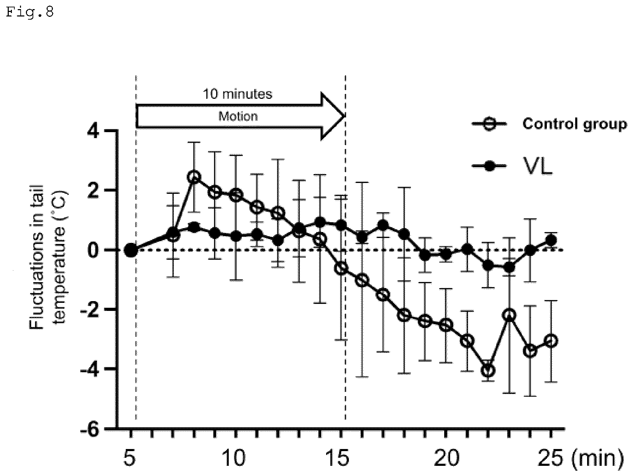

Figure 8 is a graph showing the fluctuations in the tail temperature of inner ear-damaged mice and normal mice. As shown in the graph in Figure 7, the mice with intact inner ears (control group) exhibited an increase in tail temperature during the motion stimulus, followed by a decrease after the stimulus. In contrast, the inner ear-damaged mice (VL) showed little change in tail temperature both during and after the motion stimulus. These results indicate that the vestibular function in the inner ear plays a crucial role in the onset of motion sickness after the motion stimulus.

As mentioned above, after confirming the correlation between the decrease in tail temperature and the onset of motion sickness, as well as the relationship between vestibular function and motion sickness, the present inventors conducted two experiments on mice to verify the effectiveness of pure tone stimulus in alleviating motion sickness, as described in the conventional technology. In the first experiment, the effect of pure tone stimulus applied before the motion stimulus on the improvement of motion sickness was examined. Specifically, six mice were prepared, and three mice (pre-motion pure tone stimulus group) were exposed to a 5-minute pure tone stimulus with a sound pressure level of 85 dBZ and a frequency of 100 Hz before receiving a 10-minute motion stimulus. Meanwhile, the other three mice (control group) were given the 10-minute motion stimulus without any sound stimulus.

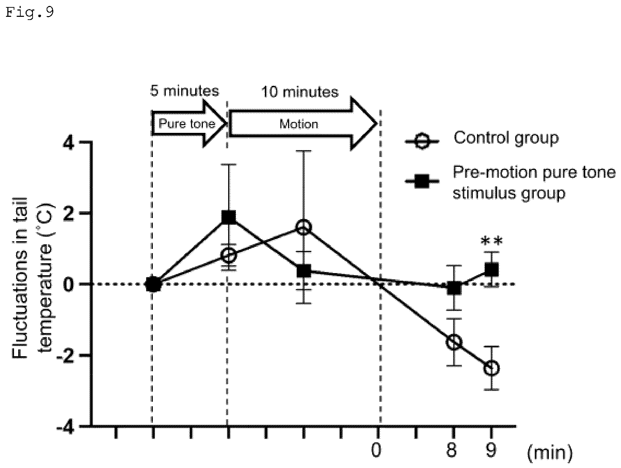

Figure 9 is a graph showing the fluctuations in tail temperature for the control group and the pre-motion pure tone stimulus group. The control group, which did not receive pure tone stimulus, showed an increase in tail temperature during the motion stimulus, followed by a decrease after the stimulus, suggesting that motion sickness was induced. In contrast, the pre-motion pure tone stimulus group, which received pure tone stimulus before the motion stimulus, showed little change in tail temperature both during and after the motion stimulus, suggesting that motion sickness was not induced. Therefore, it is inferred that pure tone stimulus given before the motion stimulus leads to an improvement effect (prevention of onset) for motion sickness.

In the second experiment, the effect of pure tone stimulus administered after the motion stimulus on alleviating motion sickness was examined. Specifically, six mice were prepared. Three mice (post-motion pure tone stimulus group) were subjected to a 10-minute motion stimulus, followed by a 5-minute pure tone stimulus with a sound pressure level of 85 dBZ and a frequency of 100 Hz. Meanwhile, the other three mice (control group) were given the 10-minute motion stimulus but did not receive any subsequent pure tone stimulus.

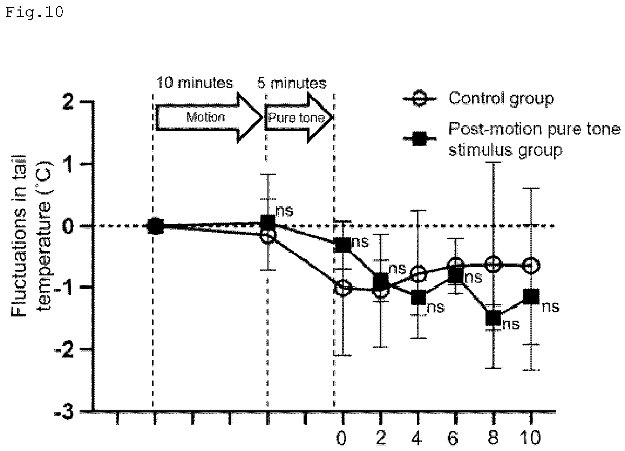

Figure 10 is a graph showing the fluctuations in tail temperature for the control group and the post-motion pure tone stimulus group. In the control group, which did not receive pure tone stimulus, the tail temperature decreased after the motion stimulus, suggesting that motion sickness was induced. Similarly, in the post-motion pure tone stimulus group, which received pure tone stimulus after the motion stimulus, the tail temperature also decreased after the motion stimulus, suggesting that motion sickness was induced. These results indicate that the effect of pure tone stimulus in alleviating motion sickness may be somewhat limited when applied after the motion stimulus.

As shown in Example 6 described later, mice that received pure tone stimulus during the motion stimulus exhibited a slight improvement in motion sickness compared to mice that did not receive sound stimulus during the motion stimulus. However, the improvement effect was not as pronounced as when pure tone stimulus was administered prior to the motion stimulus.

Thus, in the conventional technology, it is preferable to administer sound stimulus before the motion stimulus. However, when it is not possible to predict in advance the likelihood of being exposed to a level of motion stimulus that requires prevention, the conventional technology may be insufficient to address the issue.

The present disclosure has been devised to address the above-mentioned problems and aims to further improve vestibular function.

### Solution to Problem

In light of the above challenges, the present inventors have conducted extensive research and discovered that varying the frequency of sound stimulus can resolve these issues. Specifically, the present disclosure encompasses the following embodiments.

### Clause 1.

A vestibular function improvement device that generates a sound stimulus to improve the vestibular function of the inner ear,
wherein the frequency of the sound stimulus changes over time.

### Clause 2.

The vestibular function improvement device according to clause 1, wherein the frequency is 120 Hz or lower.

### Clause 3.

The vestibular function improvement device according to clause 1, wherein the difference between the maximum and minimum values of the frequency is 60 Hz or less.

### Clause 4.

The vestibular function improvement device according to any one of clauses 1 to 3, wherein the frequency changes periodically.

### Clause 5.

The vestibular function improvement device according to any one of clauses 1 to 3, wherein the frequency changes continuously.

### Clause 6.

The vestibular function improvement device according to any one of clauses 1 to 3, wherein the frequency changes with a cycle time of 0.08 seconds to 13 seconds.

### Clause 7.

The vestibular function improvement device according to any one of clauses 1 to 3, comprising a setting unit configured to set the timing for generating the sound stimulus,
wherein the setting unit is configured to detect that a subject is receiving a motion sickness stimulus and to generate the sound stimulus while the motion sickness stimulus is being applied to the subject.

### Clause 8.

The vestibular function improvement device according to clause 1, wherein the sound stimulus is a sound stimulus for improving motion sickness.

### Clause 9.

The vestibular function improvement device according to clause 8, wherein the sound stimulus is a sound stimulus for improving motion sickness caused by a visual stimulus received from images or similar sources without accompanying motion.

### Clause 10.

A motion sickness stimulus application system for providing a motion sickness stimulus to a subject, comprising:
a vestibular function improvement device that generates a sound stimulus to improve the vestibular function in the inner ear of the subject,
wherein the frequency of the sound stimulus changes over time.

### Clause 11.

The motion sickness stimulus application system according to clause 10, comprising: a setting unit configured to set the timing for generating the sound stimulus
wherein the setting unit is configured to generate the sound stimulus while the motion sickness stimulus is being applied to the subject.

### Clause 12.

A program for causing a computer to implement a function of generating a sound stimulus to improve vestibular function,
wherein the frequency of the sound stimulus changes over time.

### Effect of the Invention

According to the present disclosure, vestibular function can be further improved. For example, the present disclosure enables the improvement of motion sickness even when a sound stimulus are applied during motion, thereby further expanding the range of applications for societal implementation.

### BRIEF DESCRIPTION OF THE DRAWINGS

Figure 1(a) is a block diagram illustrating the configuration of a vestibular function improvement device 1 according to an embodiment of the present disclosure.
Figure 1(b) is a side view showing the configuration of the vestibular function improvement device 1.
Figure 1(c) is a plan view showing the configuration of the vestibular function improvement device 1.
Figure 2(a) is a graph showing an example of the waveform of the sound stimulus.
Figure 2(b) is a graph showing a variation of the waveform of the sound stimulus.
Figure 3(a) is a graph showing the fluctuations in tail temperature after motion stimulus in cases where no sound stimulus was applied and where a pure tone stimulus was applied in advance.
Figure 3(b) is a graph showing the fluctuations in tail temperature after motion stimulus in cases where no sound stimulus was applied and where a sweep tone stimulus was applied in advance.
Figure 4(a) is a graph showing fluctuations in tail temperature in mice when no sound stimulus was applied during the motion stimulus and when a pure tone stimulus was applied during the motion stimulus.
Figure 4(b) is a graph showing fluctuations in tail temperature in mice when no sound stimulus was applied during the motion stimulus and when a sweep tone stimulus was applied during the first half of the motion stimulus.
Figure 5(a) is a graph showing fluctuations in tail temperature in mice when no sound stimulus was applied during the motion stimulus and when a sweep tone stimulus was applied during the latter half of the motion stimulus.
Figure 5(b) is a graph showing fluctuations in tail temperature in mice when no sound stimulus was applied during the motion stimulus and when a sweep tone stimulus was applied after the motion stimulus.
Figure 6(a) is a graph showing fluctuations in tail temperature in mice when no sound stimulus was applied during the motion stimulus and when a sweep tone with a frequency varying in the range of 40-80 Hz was applied during the motion stimulus.
Figure 6(b) is a graph showing fluctuations in tail temperature in mice when no sound stimulus was applied during the motion stimulus and when a sweep tone with a frequency varying in the range of 80-120 Hz was applied during the motion stimulus.
Figure 6(c) is a graph showing fluctuations in tail temperature in mice when no sound stimulus was applied during the motion stimulus and when a sweep tone with a frequency varying in the range of 120-160 Hz was applied during the motion stimulus.
Figure 7 is a graph showing fluctuations in the tail temperature in mice during the application of a motion stimulus.
Figure 8 is a graph showing fluctuations in the tail temperature in inner ear-damaged mice and normal mice.
Figure 9 illustrates the motion sickness improvement effects of a sound stimulus in conventional techniques, showing graphs of tail temperature fluctuations in the control group and the pre-motion pure-tone stimulus group.
Figure 10 illustrates the motion sickness improvement effects of a sound stimulus in conventional techniques, showing graphs of tail temperature fluctuations in the control group and the post-motion pure-tone stimulus group.
Figures 11(a) represents typical data of postural sway for one subject in the center of gravity sway test.
Figures 11(b) represents typical data of postural sway for one subject in the center of gravity sway test.
Figures 11(c) represents typical data of postural sway for one subject in the center of gravity sway test.
Figure 12 is a box plot showing the deterioration rate of postural sway after a motion stimulus compared to before the motion stimulus.

### Description of Embodiments

The following describes the embodiments of the present disclosure with reference to the accompanying drawings. It should be noted that the present disclosure is not limited to the embodiments described below, and various modifications can be made as long as they do not depart from the spirit of the disclosure.

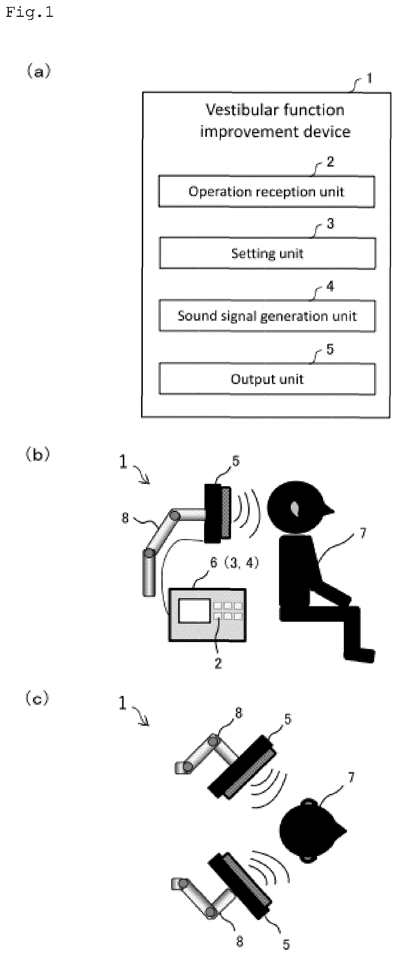

Figure 1(a) is a block diagram illustrating the configuration of a vestibular function improvement device 1 according to the present embodiment, and Figures 1(b) and 1(c) are a side view and a plan view, respectively, showing the configuration of the vestibular function improvement device 1. The vestibular function improvement device 1 has a function to generate sounds based on patterns of a sound stimulus designed to improve vestibular function, particularly a sound stimulus capable of alleviating motion sickness even when provided during motion. Hereinafter, the sounds generated by the vestibular function improvement device 1 are referred to as "a sound stimulus".

The vestibular function improvement device 1 includes an operation reception unit 2 that receives operations for outputting a sound stimulus, a setting unit 3 that sets the timing for generating the sound stimulus in response to the operation, a sound signal generation unit 4 that generates a signal for the set sound stimulus, and an output unit 5 that outputs the sound stimulus based on the generated signal.

The operation reception unit 2 is, for example, composed of an operation button. The setting unit 3 and the sound signal generation unit 4 are built into the main body 6. The sound signal generation unit 4 has the function of generating a signal for a sound stimulus with a frequency that temporally changes, either continuously or intermittently.

The output unit 5 is a speaker that outputs sound from the vestibular function improvement device 1 to the outside. In this embodiment, as shown in Figures 1(b) and 1(c), the output unit 5 is held by a movable arm 8 at the right rear diagonal and left rear diagonal positions of the head of a subject 7. The output unit 5 may also output sound from headphones or earphones connected to the vestibular function improvement device 1.

It is preferable that the setting unit 3 controls the generation of a sound stimulus during the period when a motion sickness stimulus (that induces motion sickness in the subject 7, including a motion stimulus and a visual stimulus via images) is applied to the subject 7. This ensures that the sound stimulus is not generated before or after the motion sickness stimulus, enabling efficient prevention or improvement of motion sickness. Furthermore, the setting unit 3 may have a function to detect when the subject 7 is receiving the motion sickness stimulus (detection is not limited to the use of sensing but may also involve estimation based on the subject's operation).

In Figure 1, each element described as a functional block performing various processes can be implemented in hardware as circuit blocks, memory, and other LSI components, or in software as programs loaded into memory. Therefore, it will be understood by those skilled in the art that these functional blocks can be realized in various forms using hardware alone, software alone, or a combination of both, and are not limited to any one of these configurations.

As will be described in more detail later, the vestibular function improvement device 1 generates a sound stimulus in which the frequency changes over time for improving vestibular function. The sound stimulus may be a continuous sound that is emitted continuously over time, or it may be a discontinuous sound (intermittent sound) emitted intermittently and/or periodically.

The vestibular function improvement device 1 may be installed as a medical device in medical facilities such as hospitals, or it may be marketed as a health device for improving the vestibular function and promoting health in healthy individuals. It is preferable for the vestibular function improvement device 1 to be compactly designed for portability.

In this disclosure, "improving the vestibular function of the inner ear" means that there is a significant difference (for example, a p-value of less than 0.05) in an indicator of the degree of vestibular function (such as motion sickness) between each of a living body that has been given the sound stimulus and one that has not been given the sound stimulus. Examples of such indicators include tail temperature variations in mice, and the deterioration rate of postural sway before and after a motion stimulus in humans.

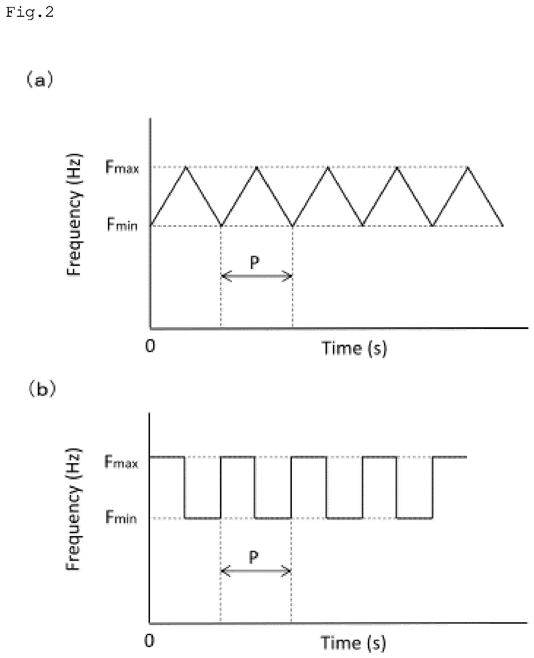

The sound stimulus generated by the vestibular function improvement device 1 is a sound stimulus with a frequency that changes over time. Figure 2(a) is a graph showing an example of the waveform of the sound stimulus. This sound stimulus has a frequency that periodically changes with a cycle time P, but the frequency change does not have to be periodic. The frequency is not specifically limited as long as it is within the audible range (above 20 Hz), but it is preferable to be below 140 Hz. Additionally, the range of the frequency, that is, the difference between the maximum value F max and the minimum value F min, is not specifically limited, but it is preferable to be 60 Hz or less.

Additionally, the sound stimulus shown in Figure 2(a) has a frequency that changes continuously (gradually), and such a sound stimulus is hereinafter referred to as a "sweep sound." The change in the frequency of the sweep sound does not have to be linear.

Additionally, as shown in Figure 2(b), the frequency of the sound stimulus may change discontinuously.

Furthermore, the sound pressure level of the sound stimulus should be at a level that is noticeable to humans, preferably 80 dBZ or higher, and more preferably 85 dBZ or higher. In this disclosure, "sound pressure level" refers to the sound pressure level without any hearing correction. Also, when the subject is a human, the sound pressure level refers to the sound pressure level at the ear, but when the subject is a mouse, it refers to the sound pressure level at the location of the mouse.

Additionally, in the vestibular function improvement device 1 shown in Figure 1, the sound stimulus can be set by the user's operation, but it may also be configured such that the sound stimulus cannot be set. For example, the vestibular function improvement device may be configured to output only one type of sound stimulus by playing prestored sound data. Alternatively, the vestibular function improvement device may be realized by inputting sound data for the sound stimulus to improve vestibular function into a general-purpose audio device, smartphone, or other computer via an electrical communication line or a storage medium such as a flash memory.

### Examples

The present inventors have discovered through experiments and the like, as described below, that sounds with frequencies that change over time are sound stimuli capable of improving vestibular function, and in particular, that these sound stimuli can improve motion sickness not only when applied before motion but also during motion.

### Example 1

In Example 1, the effect of motion sickness improvement was examined by providing a sound stimulus before motion, using a sweep tone as well as a pure tone. Specifically, three mice were prepared, and a motion stimulus was applied for 10 minutes without any sound stimulus. After a time interval, a pure tone stimulus with a sound pressure level of 85 dBZ at a frequency of 100 Hz was applied to the same mice for 5 minutes in advance, followed by a motion stimulus for 10 minutes. During the experiment, the tail temperature of each mouse was measured using a thermography.

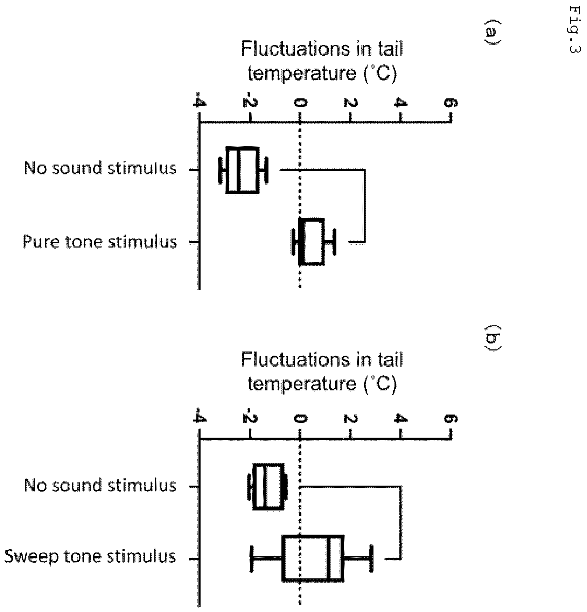

Figure 3(a) is a graph showing the variation in tail temperature after a motion stimulus in cases where no sound stimulus was provided and where a pure tone stimulus was provided in advance. Similar to the results shown in Figure 9, providing a pure tone stimulus in advance significantly suppressed the decrease in temperature after the motion stimulus, demonstrating an improvement effect on motion sickness.

An experiment on motion was conducted using a sweep tone. Specifically, three mice were prepared, and a motion stimulus was applied for 10 minutes without any sound stimulus. After a time interval, the same mice were exposed to a sweep tone stimulus with a sound pressure level of 85 dBZ, where the frequency ranged from 60 to 110 Hz and changed over a cycle time of 0.1 seconds, for 5 minutes in advance, followed by a motion stimulus for 10 minutes.

Figure 3(b) is a graph showing the variations in tail temperature after a motion stimulus in cases where no sound stimulus was provided and where a sweep tone stimulus was provided in advance. When the sweep tone stimulus was provided in advance, the decrease in temperature after the motion stimulus was significantly suppressed compared to when no sound stimulus was provided, demonstrating a motion sickness improvement effect.

From the above results, it was found that providing a sweep tone stimulus before motion has a motion sickness improvement effect.

### Example 2

In Example 2, the effect of motion sickness improvement was examined by providing a pure tone and a sweep tone to mice during motion. Specifically, six mice were prepared, and a motion stimulus was applied for 10 minutes without any sound stimulus. After a time interval, the same mice were subjected to a motion stimulus for 10 minutes, during which a pure tone stimulus with a sound pressure level of 85 dBZ and a frequency of 100 Hz was applied for the first 5 minutes from the start of the motion stimulus. Next, after another time interval, the same mice were subjected to a motion stimulus for 10 minutes, with a sweep tone stimulus (sound pressure level: 85 dBZ; frequency: 80-120 Hz; cycle time: 0.1 seconds) applied during the first half of the motion stimulus (0-5 minutes). Then, after another time interval, the same mice were subjected to a motion stimulus for 10 minutes, with the sweep tone stimulus (sound pressure level: 85 dBZ; frequency: 80-120 Hz; cycle time: 0.1 seconds) applied during the second half of the motion stimulus (5-10 minutes). Finally, after another time interval, the same mice were subjected to a motion stimulus for 10 minutes, followed by the sweep tone stimulus (sound pressure level: 85 dBZ; frequency: 80-120 Hz; cycle time: 0.1 seconds) applied for 5 minutes, starting 5 minutes after the end of the motion stimulus. During all experiments, the tail temperature of each mouse was measured using thermography.

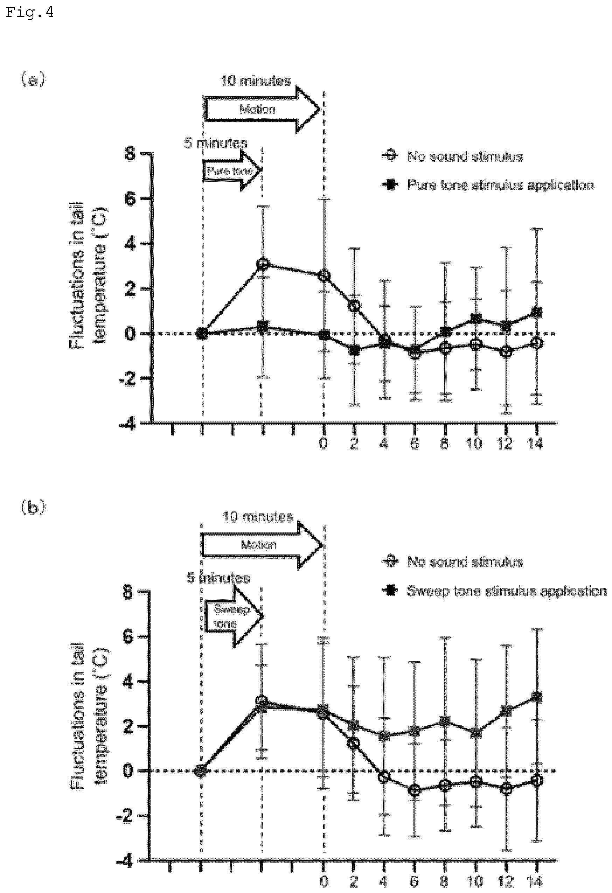

Figure 4(a) is a graph showing variations in the tail temperature of mice when no sound stimulus was applied during a motion stimulus and when a pure tone stimulus was applied during a motion stimulus. No significant difference in temperature variations after the motion stimulus was observed between the two cases, indicating that applying a pure tone stimulus during the motion stimulus had little effect in improving motion sickness.

Figure 4(b) is a graph showing variations in the tail temperature of mice when no sound stimulus was applied during a motion stimulus and when a sweep tone stimulus was applied during the first half of the motion stimulus. In the case where the sweep tone stimulus was applied, the decrease in temperature after the motion stimulus was significantly suppressed compared to when no sound stimulus was applied, demonstrating an improvement in motion sickness.

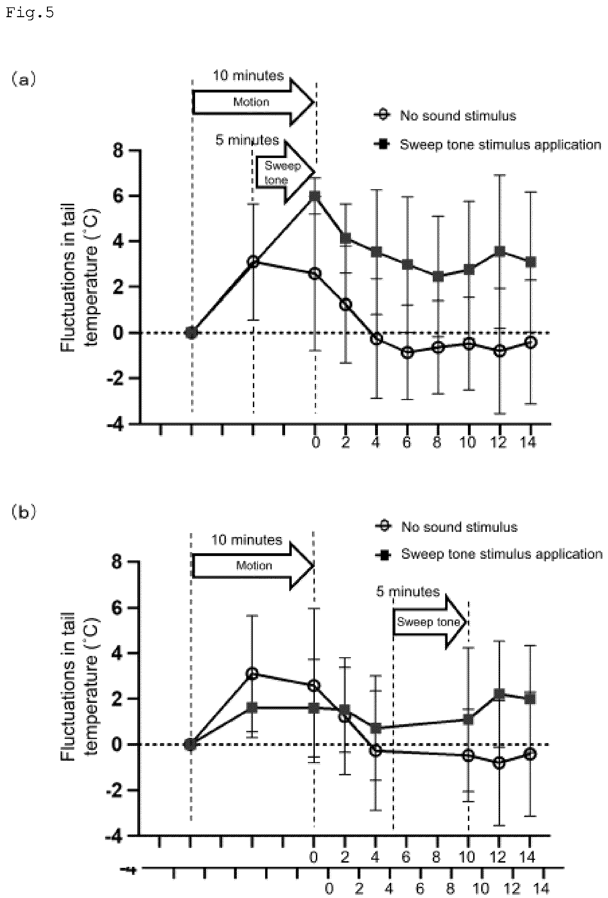

Figure 5(a) is a graph showing variations in the tail temperature of mice when no sound stimulus was applied during a motion stimulus and when a sweep tone stimulus was applied during the latter half of the motion stimulus. In the case where the sweep tone stimulus was applied, the decrease in temperature after the motion stimulus was significantly suppressed compared to when no sound stimulus was applied, demonstrating an improvement in motion sickness.

Figure 5(b) is a graph showing variations in the tail temperature of mice when no sound stimulus was applied during a motion stimulus and when a sweep tone stimulus was applied for 5 minutes, starting 5 minutes after the end of the motion stimulus. In the case where the sweep tone stimulus was applied, the decrease in temperature after the motion stimulus was significantly suppressed compared to when no sound stimulus was applied, demonstrating an improvement in motion sickness.

From the above results, it was found that providing a sweep tone stimulus during motion can improve motion sickness. In other words, it was found that the vestibular function improvement effect of a sweep tone stimulus is greater than that of a pure tone stimulus.

### Example 3

In Example 3, the effect of motion sickness improvement was examined by providing sweep tones with different frequency ranges (the difference between the maximum and minimum frequencies) to mice during motion. Specifically, six mice were prepared, and a motion stimulus was applied for 10 minutes without any sound stimulus. After a time interval, a motion stimulus was applied to the same mice for 10 minutes, during which a sweep tone stimulus with a sound pressure level of 85 dBZ and a frequency range of 40-80 Hz, changing at a cycle time of 0.1 seconds, was applied. Next, after another time interval, a motion stimulus was applied to the same mice for 10 minutes, during which a sweep tone stimulus with a sound pressure level of 85 dBZ and a frequency range of 80-120 Hz, changing at a cycle time of 0.1 seconds, was applied. Then, after another time interval, a motion stimulus was applied to the same mice for 10 minutes, during which a sweep tone stimulus with a sound pressure level of 85 dBZ and a frequency range of 120-160 Hz, changing at a cycle time of 0.1 seconds, was applied. During the experiments, the tail temperature of each mouse was measured using thermography.

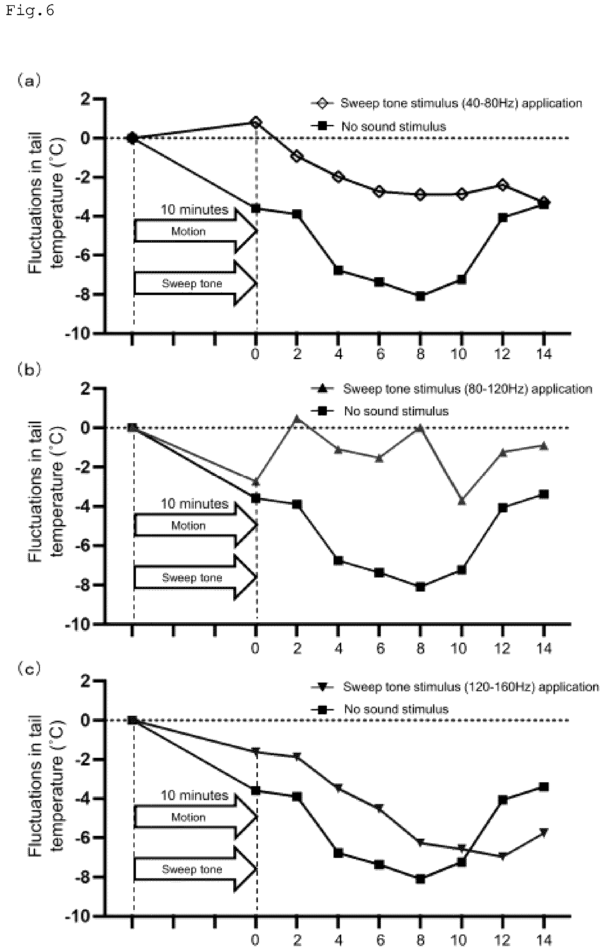

Figure 6(a) is a graph showing fluctuations in tail temperature of mice in the case where no sound stimulus was provided during the motion stimulus and in the case where a sweep tone with a frequency range of 40-80 Hz was provided during the motion stimulus. When the sweep tone stimulus with a frequency range of 40-80 Hz was provided, the post-motion temperature drop was significantly suppressed compared to when no sound stimulus was provided, demonstrating an improvement in motion sickness.

Figure 6(b) is a graph showing fluctuations in tail temperature of mice in the case where no sound stimulus was provided during the motion stimulus and in the case where a sweep tone with a frequency range of 80-120 Hz was provided during the motion stimulus. When the sweep tone stimulus with a frequency range of 80-120 Hz was provided, the post-motion temperature drop was also significantly suppressed compared to when no sound stimulus was provided, demonstrating an improvement in motion sickness.

Figure 6(c) is a graph showing fluctuations in tail temperature of mice in the case where no sound stimulus was provided during the motion stimulus and in the case where a sweep tone with a frequency range of 120-160 Hz was provided during the motion stimulus. When the sweep tone stimulus with a frequency range of 120-160 Hz was provided, no significant difference in post-motion temperature changes was observed compared to when no sound stimulus was provided, indicating that no improvement in motion sickness was demonstrated.

From the above results, it was found that the frequency of the sweep tone is preferably 120 Hz or lower.

### Example 4

In Example 4, a postural sway test was conducted on a healthy human subject to verify the effect of motion sickness improvement through sweep tone stimulus. Specifically, for one subject, a rotational motion stimulus (12 rpm) was applied for 1 minute without any sound stimulus, and postural sway (eyes closed for 1 minute) was measured before and after the motion stimulus. After a time interval, the same subject was exposed to the same motion stimulus for 1 minute while being provided with a pure tone stimulus with a sound pressure level of 85 dBZ and a frequency of 100 Hz via headphones, and postural sway was measured before and after the motion stimulus. Next, after another time interval, the same subject was exposed to the same motion stimulus for 1 minute while being provided with a sweep tone stimulus via headphones. The sweep tone had a sound pressure level of 85 dBZ and a frequency range of 90-110 Hz, changing at a cycle time of 0.1 seconds. Postural sway was measured before and after the motion stimulation.

Table 1 shows the deterioration rate of postural sway after the motion stimulation compared to before the motion stimulation.

**Table 1**

| No sound stimulus | Pure tone stimulus | Sweep tone stimulus |
|---|---|---|
| 1.5 | 1.5 | 1.1 |

Even when a pure tone stimulus was applied, the deterioration rate of postural sway remained unchanged compared to when no sound stimulus was applied, indicating that the motion sickness improvement effect was minimal. In contrast, when a sweep tone stimulus was applied, the deterioration rate of postural sway decreased, demonstrating an improvement in motion sickness.

Thus, in experiments conducted on humans, it was found that providing a sweep tone stimulus during motion stimuli has a motion sickness improvement effect. In other words, the vestibular function improvement effect of a sweep tone stimulus is greater than that of a pure tone stimulus.

### Example 5

In Example 5, vestibular function in humans was evaluated using a VEMP (vestibular evoked myogenic potential) test. Specifically, one subject underwent the first VEMP test, followed by a 5-minute rest. After the rest, a sweep tone stimulus with a sound pressure level of 80 dBZ and a frequency range of 95-105 Hz, changing at a cycle time of 0.1 seconds, was applied via headphones for 1 minute. Immediately after this, the second VEMP test was conducted. As a result, the potential difference (cVEMP amplitude) in the first VEMP test was 51.82 µV, while the potential difference in the second VEMP test increased to 92.90 µV, resulting in a difference of 41.08 µV. Since an improvement in vestibular function leads to an increase in the amplitude of the potential difference, it was concluded that applying a sweep tone stimulus provides an excellent vestibular function improvement effect.

When a pure tone at 100 Hz was applied for 1 minute, the potential difference was 4.12 µV. The sweep tone was found to be nearly 10 times more effective than the pure tone, demonstrating a significantly superior vestibular function improvement effect.

Since VEMP tests and postural sway tests are used to evaluate vestibular and balance functions, the results of Examples 4 and 5 suggest that the present disclosure is also effective for addressing conditions caused by the decline of vestibular and balance functions, such as physical discomfort, motion sickness (e.g., car sickness), space motion sickness, vertigo, locomotive syndrome, as well as for preventing falls and fall-related accidents.

### Example 6

In Example 6, the vestibular function of mice was evaluated using a balance beam test. Specifically, 15 ICR (Institute of Cancer Research) mice (6 months old, male) were subjected to a linear motion stimulus (horizontal: 80 rpm, vertical: 50 rpm) for 15 minutes. Five mice (non-stimulus group) were not provided with any sound stimulus during the motion stimulus. Another five mice (pure tone stimulus group) were given a pure tone stimulus with a sound pressure level of 85 dBZ and a frequency of 100 Hz for 5 minutes from the start of the motion stimulus. The remaining five mice (sweep tone stimulus group) were given a sweep tone stimulus with a sound pressure level of 85 dBZ and a frequency range of 90-110 Hz, changing at a cycle time of 0.1 seconds, for 5 minutes from the start of the motion stimulus. After the motion stimulus, the balance beam test was conducted five times consecutively for each mouse to evaluate motion sickness. If a mouse fell even once during the five trials, it was counted as part of the "number of mice that fell."

The results of the balance beam test are shown in Table 2.

**Table 2**

| Non-Stimulus Group | Pure tone stimulus group | Sweep tone stimulus group |
|---|---|---|
| 4/5 | 2/5 | 0/5 |

In the non-stimulus group, 4 out of 5 mice fell, while in the pure tone stimulus group, 2 out of 5 mice fell. In contrast, none of the mice in the sweep tone stimulus group fell. These results indicate that providing a sweep tone stimulus during the motion stimulus has a significant motion sickness improvement effect. It was also observed that providing a pure tone stimulus during the motion stimulus resulted in a slight improvement in motion sickness.

In the above Examples 1 to 3, the improvement effect on motion sickness was indirectly evaluated by measuring the tail temperature of mice. However, in this example, the vestibular function was measured using the balance beam test, allowing for a direct evaluation of the improvement effect on motion sickness. Therefore, this example more clearly demonstrates that the motion sickness improvement effect of the sweep tone stimulus is greater than that of the non-stimulus and pure tone stimulus.

### Example 7

In Example 7, a postural sway test was conducted on healthy human subjects to verify the motion sickness improvement effect of sweep tone stimulus. The test followed the same protocol as in Example 4 and was conducted on seven healthy individuals (in their 20s; six males and one female).

The Romberg test, one of the postural sway tests, involves standing upright with both feet together and measuring the center of gravity trajectory for 60 seconds with eyes open and for 60 seconds with eyes closed. In the Romberg test, the trajectory length Romberg ratio, represented by (trajectory length with eyes closed / trajectory length with eyes open), and the perimeter area Romberg ratio, represented by (area of trajectory with eyes closed / area of trajectory with eyes open), are used as evaluation indicators.

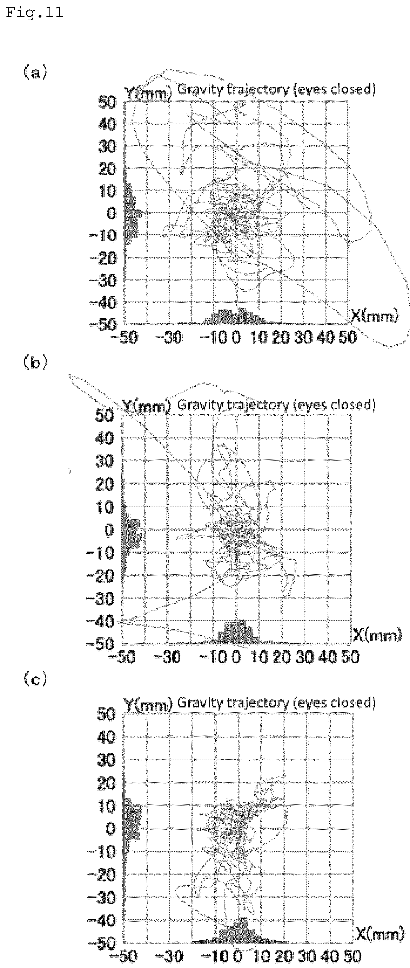

Figures 11(a) to (c) represent typical data on postural sway from one subject in the postural sway test. Specifically, Figure 11(a) shows the postural sway data when no sound stimulus was provided during the motion stimulus, Figure 11(b) shows the postural sway data when the above pure tone stimulus was provided during the motion stimulus, and Figure 11(c) shows the postural sway data when the above sweep tone stimulus was provided during the motion stimulus. It can be seen that providing the sweep tone stimulus effectively suppresses the worsening of postural sway.

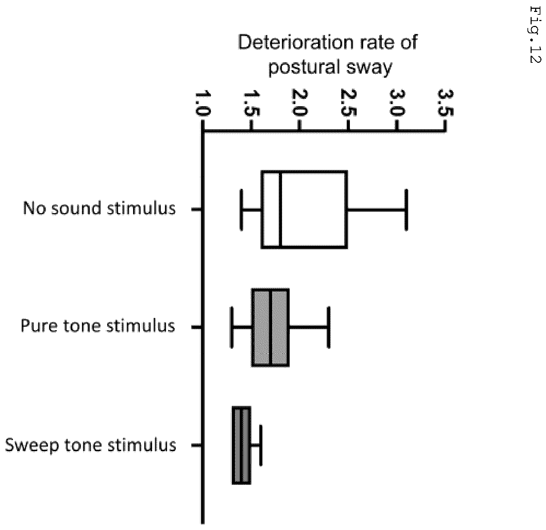

Figure 12 is a box plot showing the deterioration rate of postural sway after the motion stimulus compared to before the motion stimulus. Providing a pure tone stimulus was found to suppress the worsening of postural sway compared to when no sound stimulus was provided. Furthermore, providing a sweep tone stimulus was found to suppress the worsening of postural sway even more effectively than providing a pure tone stimulus.

In this example, experiments were conducted using 100 Hz sound stimuli (100 Hz pure tone and sweep tone with a center frequency of 100 Hz). It has been found that similar trends are observed with a sound stimulus having a frequency of 300 Hz or lower. Among these, sweep tones with a center frequency of 150 Hz or lower are particularly preferred, and sweep tones with a center frequency of 120 Hz or lower are even more preferred. Furthermore, a sweep tone stimulus that changes with a cycle time of 0.08 s to 13 s has also been shown to improve motion sickness in humans, with a more preferred cycle time being 0.08 seconds to 3 seconds.

Furthermore, significant improvement effects on motion sickness induced by a non-motion visual stimulus (such as VR sickness and XR sickness) were confirmed through experiments on 14 individuals. Therefore, it can be concluded that the vestibular function improvement device in this example is also effective in improving motion sickness caused by a visual stimulus presented through non-motion images.

### Additional Notes

The embodiments and examples of the present disclosure have been described above; however, the present disclosure is not limited to these embodiments and examples, and various modifications can be made within the scope indicated in the claims. For example, while the above embodiments and examples provide specific numerical values for frequencies and other parameters of the sound stimulus with time-varying frequency, such numerical values are not particularly limited as long as there is a significant difference between the indicators of the degree of vestibular function in a living body subjected to the sound stimulus and those in a living body not subjected to the sound stimulus.

### Industrial Applicability

The vestibular function improvement device of the present disclosure can prevent or alleviate motion sickness in a subject by being installed in a motion stimulus application system that provides a subject with a motion sickness stimulus (including a motion stimulus and a visual stimulus through images) that induces motion sickness. This system does not primarily aim to provide a subject with a motion sickness stimulus but may incidentally do so, the system including vehicles like cars and ships that cause such a stimulus for a subject riding thereon. Specifically, the vestibular function improvement device can be installed on the seat of a vehicle. Additionally, it may be installed on devices used by a subject while experiencing a visual stimulus through images, such as monitors, chairs, controllers, or glasses used for viewing VR content.

### Description of Reference Numerals

- 1: Vestibular function improvement device
- 2: Operation reception unit
- 3: Setting unit
- 4: Sound signal generation unit
- 5: Output unit
- 6: Main body
- 7: Subject
- 8: Movable arm

## Claims

1. A vestibular function improvement device that generates a sound stimulus to improve the vestibular function of the inner ear,
wherein the frequency of said sound stimulus changes over time.

2. The vestibular function improvement device according to claim 1, wherein said frequency is 120 Hz or lower.

3. The vestibular function improvement device according to claim 1, wherein the difference between the maximum and minimum values of said frequency is 60 Hz or less.

4. The vestibular function improvement device according to any one of claims 1 to 3, wherein said frequency changes periodically.

5. The vestibular function improvement device according to any one of claims 1 to 3, wherein said frequency changes continuously.

6. The vestibular function improvement device according to any one of claims 1 to 3, wherein said frequency changes with a cycle time of 0.08 seconds to 13 seconds.

7. The vestibular function improvement device according to any one of claims 1 to 3, comprising: a setting unit configured to set the timing for generating said sound stimulus,
wherein said setting unit is configured to detect that a subject is receiving a motion sickness stimulus and to generate said sound stimulus while said motion sickness stimulus is being applied to the subject.

8. The vestibular function improvement device according to claim 1, wherein said sound stimulus is a sound stimulus for improving motion sickness.

9. The vestibular function improvement device according to claim 8, wherein said sound stimulus is a sound stimulus for improving motion sickness caused by a visual stimulus without accompanying motion.

10. A motion sickness stimulus application system for providing a motion sickness stimulus to a subject, comprising:
a vestibular function improvement device that generates a sound stimulus to improve the vestibular function in the inner ear of said subject,
wherein the frequency of the sound stimulus changes over time.

11. The motion sickness stimulus application system according to claim 10, comprising: a setting unit configured to set the timing for generating the sound stimulus,
wherein said setting unit is configured to generate said sound stimulus while said motion sickness stimulus is being applied to said subject.

12. A program for causing a computer to implement a function of generating a sound stimulus to improve vestibular function,
wherein the frequency of said sound stimulus changes over time.
